# EUROPEAN PATENT SPECIFICATION

(11) **EP 1 542 759 B1**
(45) Date of publication and mention of the grant of the patent: **03.12.2014**
(21) Application number: 03791935.4
(22) Date of filing: 28.08.2003
(51) Int. Cl.: A61M 25/00

(54) **DOUBLE-Y-SHAPED MULTI-LUMEN CATHETER WITH SELECTIVELY ATTACHABLE HUBS**
DOPPEL-Y-FÖRMIGER MULTILUMEN-KATHETER MIT SELEKTIV ABNEHMBAREN ANSATZSTÜCKEN
CATHÉTER À LUMIÈRES MULTIPLES EN FORME DE DOUBLE Y POURVU D'EXTREMITÉS POUVANT ÊTRE SÉLECTIVEMENT FIXÉES

(30) Priority: 30.08.2002 US 231748
(43) Date of publication of application: 22.06.2005
(73) Proprietor: Arrow International, Inc., Reading, Pennsylvania 19605 (US)
(72) Inventor: WILSON, Jon, S., Winston-Salem, NC 27104 (US); GARY, Fleming, S., Palm City, FL 34990 (US); FLEMING, Carl, M., Stuart, FL 34996 (US); CASSIDY, Kenneth, T., Mocksville, NC 27028 (US); BOYD, Ronald, D., Statesboro, GA 30458 (US)
(74) Representative: von Hirschhausen, Helge
(86) International application number: PCT/US2003/027078
(87) International publication number: WO 2004/020019

(56) References cited:
- EP-A- 0 453 234
- US-A- 4 619 643
- US-A- 4 619 643
- US-A- 5 053 023
- US-A- 5 624 413
- US-A- 5 876 366
- US-A- 5 947 953
- US-A- 5 947 953
- US-A- 6 001 079
- US-A- 6 001 079

## Description

### BACKGROUND

### 1. Field of the Invention

The present invention relates generally to medical instrumentation and more specifically to a multi-lumen catheter including y-shaped distal and proximal ends, and including selectively attachable hubs for selectively connecting the catheter to a fluid exchange device.

### 2. Description of the Prior Art

Catheters, generally, are hollow, flexible tubes for insertion into a body cavity, duct, or vessel to allow the passage of fluids or distend a passageway. Catheters are often used for temporary or long-term dialysis treatment. Dialysis treatment provides for blood to be withdrawn from the patient, purified, and then returned to the patient. Thus, in dialysis treatment, catheters are used to allow passage of a patient's blood into and out of the patient's body. For optimal performance during dialysis treatment, the catheter tips, both in-flow and outflow, should be placed in close proximity to the heart. Typically, medical personnel use either a double lumen catheter or two single lumen catheters. Both types, however, present certain deficiencies.

While double lumen catheters (e.g., U.S. Pat. No. 4,895,561) allow for a single insertion of the catheter into the desired vein, double lumen catheters typically do not permit optimal catheter tip placement. Due to differences among patients, optimal tip position varies from patient to patient. Non-optimal tip position may significantly lower flow values, resulting in less effective dialysis treatment. For current double lumen catheters, a physician must make an estimate regarding the appropriate catheter tube length prior to beginning the procedure of catheterization. Then, a subcutaneous tunnel is made from a first end, which is near the area to be catheterized, to a second end, which is the preferred end position of the hub assembly, namely, away from the neck of the patient, in order to allow for more convenient access to the dialysis treatment equipment. The catheter tube is then routed forwardly into the through the subcutaneous tunnel from the second end to the first end so that the catheter tips extend outwardly from the first end of the tunnel. Either before or after tunneling, a sheath is inserted trough the first end of the tunnel and into the area to be catheterized, and the catheter tips are inserted into the sheath and the area to be catheterized. The estimated catheter tube length and subsequent forward tunneling may result in less than optimal tip placement.

With the use of two independent single lumen catheters (e.g., U.S. Pat. No. 5,776,111 to Tesio) the problem of tip placement is addressed. The hub assembly of each catheter is removable from the tube and tip portion of the catheter, thereby allowing the catheter tip to be placed directly into the vein and advanced into the desired position. Then, the proximal end of the catheter can be reversed tunneled and trimmed to a desired length. Thereafter, the hub assembly is attached. Deficiencies, however, exist in this method of catheterization as well. One problem associated with this method is that this method requires two separate venous insertions, namely, two tunnels and two of each accessory instrument used for the procedure. Therefore, there is increased surgical time required to place two catheters, there are two wound entry sites which doubles the risk of post-surgical infection, and the two catheters together are significantly larger in diameter than one double lumen catheter.

US 5947 953 (closest prior art) describes a splittable multiple catheter assembly and methods of inserting the same. The assembly includes a splittable membrane joining a first catheter and a second catheter in order to be at least partially longitudinally split from each other.

In the disclosed apparatus and method, a multi-lumen catheter includes a selectively attachable hub assembly that allows the catheter tip to be positioned accurately within a patient's vein prior to subcutaneous tunneling. The distal end of the catheter tube is selectively attachable to the hub assembly. Accordingly, after the tips of the catheter have been accurately positioned in a patient, the other end of the catheter may be reverse tunneled under the skin of a patient. Before or after tip placement, an incision is made in the skin adjacent to the point where the protruding distal end of the catheter exits the skin. A subcutaneous tunnel is then formed having a first end at the incision and a second end exiting the skin at a point remote from the first end of the tunnel, generally as the caudal direction. A sheath dilator is inserted into the tunnel, which is partially dilated so as to accommodate a tissue in-growth stabilizing cuff. The distal end of the catheter tube is routed through the subcutaneous tunnel and the cuff seated therein, thereby stabilizing the distal portion of the catheter tube in the patient. A selectively attachable hub assembly is connected to the lumens at the distal tip of the catheter tube for subsequent connection of the catheter to a fluid exchange device, such as a dialysis machine.

While the selectively attachable hub assembly described above facilitates tunneling a multi-lumen catheter in a patient, the separable hub assembly creates the need to connect the hub to a distal end of a multi-lumen catheter tube, thereby adding an additional step to the catheter insertion/connection procedure, which increases surgical time and expense. Furthermore, the hub-catheter connection provides an additional connection which may leak or separate from the catheter tube due to external loads on the hub such as by pulling or snagging. In addition, the attachable hub assembly is a relatively complex part, which makes it difficult to manufacture and, therefore, use of the hub assembly increases the cost of the catheter itself.

Therefore, there is a need for a multi-lumen catheter that can be inserted into a patient using a reverse tunneling technique, which permits accurate placement of the tips of the catheter into the area to be catheterized and that is selectively attachable to a fluid exchange device. The improved catheter should not required an extensive hub assembly, thus making it relatively inexpensive to manufacture and easy to insert into a patient.

### SUMMARY OF THE INVENTION

A multi-lumen catheter is provided for use in hemodialysis and the like. The multi-lumen catheter includes an elongated, central, multi-lumen tube portion having a distal end and a proximal end. The central tube portion has a substantially cylindrical outer shape and is internally segmented into a plurality of lumens. A distal branch portion includes a plurality of single-lumen distal extension tubes. Each distal extension tube has a proximal first end and a distal second end. The proximal first end of each distal extension tube is connected to the distal end of the central tube portion such that the single lumen of each distal extension tube is in fluid communication with one of the plurality of lumens of the central tube portion. A proximal branch portion includes a plurality of single-lumen proximal extension tubes. Each proximal extension tube has a distal first end and a proximal second end. The distal first end of each proximal extension tube is connected to the proximal end of the central tube portion such that the single lumen of each distal extension tube is in fluid communication with one of the plurality of lumens of the central tube portion. A plurality of selectively attachable connector hubs are provided, each connector hub being configured to be selectively attachable to the distal second end of one of the distal extension and being configured for selective connection to a fluid exchange device. Each lumen of the central tube portion and the lumens of the distal and proximal extension tubes in fluid communication therewith define a flow path through the catheter. An in-growth stabilizing cuff may be affixed to an outer portion of the central tube portion. ,

The multi-lumen catheter may include a central tube portion having two lumens. In such a catheter, the distal branch portion includes two distal extension tubes, and the proximal branch portion includes two proximal extension tubes. The catheter may be arranged such that the plurality of single-lumen distal extension tubes of the distal branch portion converge to form a distal multi-lumen connecting portion which connects to the distal end of the central tube portion, and the plurality of single-lumen proximal extension tubes comprising the proximal branch portion converge to form a proximal multi-lumen connecting portion which connects to the proximal end of the central tube portion.

The central tube portion, the distal extension tubes, and the proximal extension tubes may be comprised of a fusible material, and the distal extension tubes and proximal extension tubes may be respectively fused to the distal and proximal ends of the central tube portion. The distal extension tubes may have a substantially cylindrical outer shape near their distal second ends, and the proximal multi-lumen connecting portion may also have substantially cylindrical outer shape. The proximal extension tubes may have a substantially D-shaped cross-section over at least a portion of their length. Also, the proximal extension tubes may be substantially parallel to each other in a free state, and the proximal second ends of the distal extension tubes may be longitudinally spaced from each other.

The multi-lumen catheter may further include a plurality of connector hubs for connecting the catheter to a fluid exchange device. Each connector hub may configured to be connected to the distal second end of one of the distal extension tubes, and configured for connection to a portion of a fluid exchange apparatus. Each of the proximal extension tubes may include a tube wall, and each of the proximal extension tubes may include at least one opening extending through its tube wall. Further, an external portion of at least one of the distal extension tubes may include indicia which indicates a discrete flow path through the catheter.. In one arrangement, the two proximal extension tubes have longitudinal axes which intersect at an included angle in a free state, the included angle being in a range from about 10 degrees to about 30 degrees.

A y-shaped catheter junction for a multi-lumen catheter is also provided. The y-shaped junction includes a dual-lumen trunk, having a substantially cylindrical outer wall, a first end, a second end, a first lumen, and a second lumen. A first single-lumen extension tube is connected to the first end of the trunk, such that the single lumens of the first single-lumen extension tube is in fluid communication with the first lumen of the trunk. A second single-lumen extension tube is connected to the first end of the trunk such that the single lumen of the second single-lumen extension tube is in fluid communication with the second lumen of the trunk. The y-shaped junction is arranged such that the first lumen of the trunk and the first extension tube define a first flow path, and the second lumen of the trunk and the second extension tube define a second flow path. The y-shaped catheter junction may also be arranged such that the first and second extension tubes have longitudinal axes which intersect at an included angle near the first end of the trunk in a free state, the included angle being in a range from about 10 degrees to about 30 degrees.

A method of forming a multi-lumen catheter is also disclosed. The method includes attaching a first plurality of single-lumen extension tubes to a distal end of a length of multi-lumen tubing comprising a plurality of multiple lumens, and attaching a second plurality of single-lumen extension tubes to a distal end of the length of multi-lumen tubing. Each single lumen of each extension tube is in fluid communication with one of the lumens of the length of multi-lumen tubing. The length of multi-lumen tubing may include two lumens, and the first and second pluralities of extension tubes may form substantially y-shaped junctions on each end of the length of multi-lumen tubing.

The method of forming a multi-lumen catheter may include first forming a y-shaped distal junction. The process may include providing a first length of single-lumen tubing to form a distal arterial extension tube, providing a second length of single-lumen tubing to form a distal venal extension tube, providing a first length of multi-lumen tubing comprising at least an arterial lumen and a venal lumen, and having a distal end and a proximal end, attaching an end of the distal arterial extension tube to the distal end of the first length of multi-lumen tubing such that the distal arterial extension tube is in fluid communication with the arterial lumen of the first length of multi-lumen tubing, and attaching an end of the distal venal extension tube to the distal end of the first length of multi-lumen tubing such that the distal venal extension tube is in fluid communication with the venal lumen of the first length of multi-lumen tubing. The proximal end of the first length of multi-lumen tubing forms a connecting end.

A second length of multi-lumen tubing having a distal end and a proximal end is provided. The tubing includes an arterial lumen and a venal lumen. The connecting end of the first length of multi-lumen tubing is connected to the distal end of the second length of multilumen tubing, such that the arterial extension tube of the distal junction is in fluid communication with the arterial lumen of the second length of multi-lumen tubing, and the venal extension tube of the distal junction is in fluid communication with the venal lumen of the second length of multi-lumen tubing.

A y-shaped proximal junction is also formed. This process includes providing a third length of single-lumen tubing to form a proximal arterial extension tube, and providing a fourth length of single-lumen tubing to form a proximal venal extension tube. A third length of multilumen tubing is also provided which includes at least an arterial lumen and a venal lumen, and has a distal end and a proximal end. An end of the proximal arterial extension tube is attached to the distal end of the third length of multi-lumen tubing such that the proximal arterial extension tube is in fluid communication with the arterial lumen of the third length of multi-lumen tubing. Also, an end of the proximal venal extension tube is attached to the distal end of the third length of multi-lumen tubing such that the proximal venal extension tube is in fluid communication with the venal lumen of the third length of multi-lumen tubing. The distal end of the third length of multi-lumen tubing forms an attachment end.

The attachment end of the third length of multi-lumen tubing is attached to the proximal end of the second length of multi-lumen tubing, such that the arterial extension tube of the proximal junction is in fluid communication with the arterial lumen of the second length of multi-lumen tubing, and the venal extension tube of the proximal junction is in fluid communication with the venal lumen of the second length of multi-lumen tubing. The method may also include forming at least one opening in a wall of the proximal venal extension tube, and forming at least one opening in a wall of the arterial proximal extension tube. The steps of attaching extension tubes and lengths of multi-lumen tubing together or to each other may include heat welding or similar fusing techniques. The longitudinal axes of the distal arterial extension tube and distal venal extension tube may be arranged to intersect at an included angle in a free state in a range from about 10 degrees to about 30 degrees.

A method for surgically implanting a double-y shaped multi-lumen catheter into a patient is also provided. The method is suited for implanting a multi-lumen catheter having a an elongated, central, multi-lumen tube portion, a proximal end portion including a single-lumen proximal venal extension tube and a single-lumen proximal arterial extension tube each having a proximal tip, and a distal end portion including a single-lumen distal venal extension tube and a single-lumen distal arterial extension tube each having a distal end. The method includes making an incision in the skin of the patient, and inserting the proximal tips of the proximal venal and arterial extension tubes through the incision and placing the proximal tips in the patient. A subcutaneous tunnel is formed having a first end proximate to the incision and a second end remote from the first end of the tunnel. The distal venal and arterial extension tubes and at least a portion of the central tube portion are guided through the subcutaneous tunnel such that at least the distal ends of the distal venal and arterial extension tubes extend outwardly from the tunnel through the second end of the tunnel. At least a portion of the distal end portion of the catheter is secured to the patient such as by sutures or any other suitable means.

When the catheter includes a stabilizing cuff, the method may further include dilating at least a portion of the subcutaneous tunnel to receive the cuff. Dilation of the tunnel may be accomplished by sliding a sheath dilator along the shaft of a trocar longitudinally positioned within the tunnel. The distal end portion of the catheter is secured to the patient by seating the cuff in a dilated portion of the subcutaneous tunnel.

The catheter implanting method may further include respectively connecting the distal arterial and venal extension tubes to arterial and venal legs of a fluid exchange device. Connecting the distal arterial and venal extension tubes may include connecting the distal arterial extension tube to the arterial leg with a first connector hub, and connecting the proximal venal extension tubes to the venal leg with a second connector hub. Inserting the proximal tips of the proximal venal and arterial extension tubes into a patient may include placing the proximal tip of the venal extension tube into a vein in the patient, and placing the proximal tip of the arterial extension tube into an artery in the patient.

These and other aspects of the invention will be made clear from a reading the following detailed description together with the drawings.

### BRIEF DESCRIPTION OF THE DRAWINGS

Figure 1 is a perspective view of a double y-shaped multi-lumen catheter;
Figure 2 is a cross-sectional view of a distal portion of the multi-lumen catheter of Figure 1;
Figure 3 is a partially exploded detail perspective view of a distal portion of the multilumen catheter of Figure 1;
Figure 4 is a partially exploded detail perspective view of a proximal portion of the multilumen catheter of Figure 1;
Figure 5 is a cross-sectional view of a proximal portion of the multi-lumen catheter of Figure 1;
Figures 6A-6D illustrate a procedure for tunneling a multi-lumen catheter like that of Figure 1 in a patient;
Figure 7 is a partial sectional view of a sheath dilator engaged along a trocar to dilate a portion of a subcutaneous tunnel in a patient;
Figures 8A-8C are views of a connection between a distal end of the catheter and a trocar for guiding the catheter through a subcutaneous tunnel.

### DETAILED DESCRIPTION

For the purposes of the following description and the claims appended hereto, the relative term "proximal" refers to those portions of a catheter and those portions of components of the catheter which are nearest the insertion end of the catheter, that is, the end of the catheter that is inserted into an area of a patient's body being catheterized, such as a blood vessel. Conversely, the relative term "distal" refers to those portions of a catheter and those portions of components of the catheter which are farthest from the insertion end of the catheter.

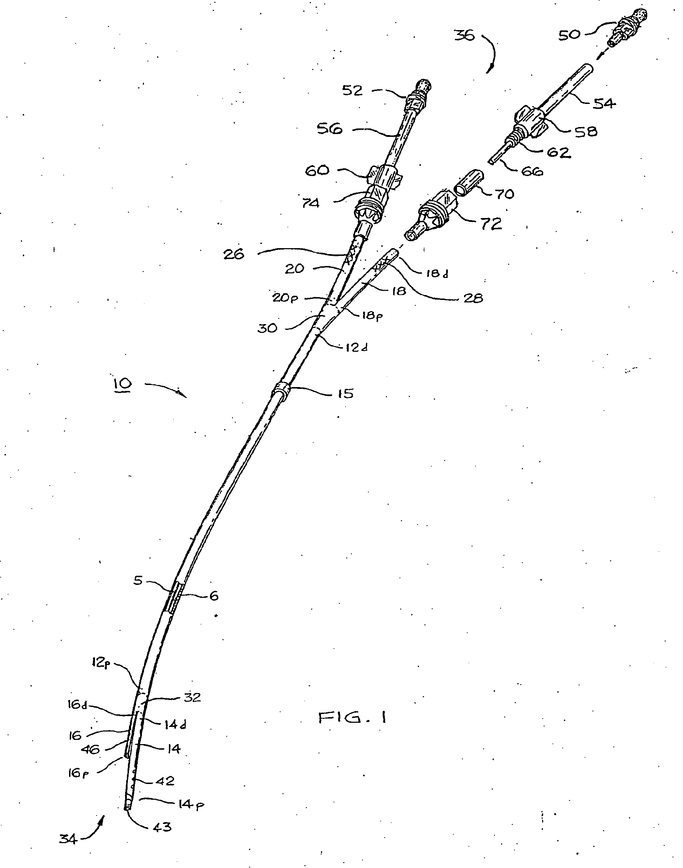

Figure 1 shows a double-Y shaped multi-lumen catheter 10 according to the present invention. The catheter 10 includes a proximal end 34 for insertion into a patient, and a distal end 36 for connection to a fluid exchange device, such as a dialysis machine or the like. The catheter 10 includes an elongated, central, multi-lumen tube portion 12, a plurality of proximal single-lumen extension tubes 14, 16, and a plurality of distal single-lumen extension tubes 18, 20. In the embodiment shown, the central tube portion 12 includes an arterial lumen 5 and a venal lumen 6. In this arrangement, the catheter 10 includes a proximal venal extension tube 14 and a distal venal extension tube 18 in fluid communication with the venal lumen 6, and a proximal arterial extension tube 16 and a distal arterial extension tube in fluid communication with the arterial lumen 5. The catheter 10 may include a stabilizing cuff 15 affixed to an outer portion of the central tube portion 12 as shown in Figure 1. Preferably, the cuff 15 is longitudinally positioned on the central tube portion 12 such that the cuff 15 will be finally positioned in a subcutaneous tunnel in a patient as described more fully below.

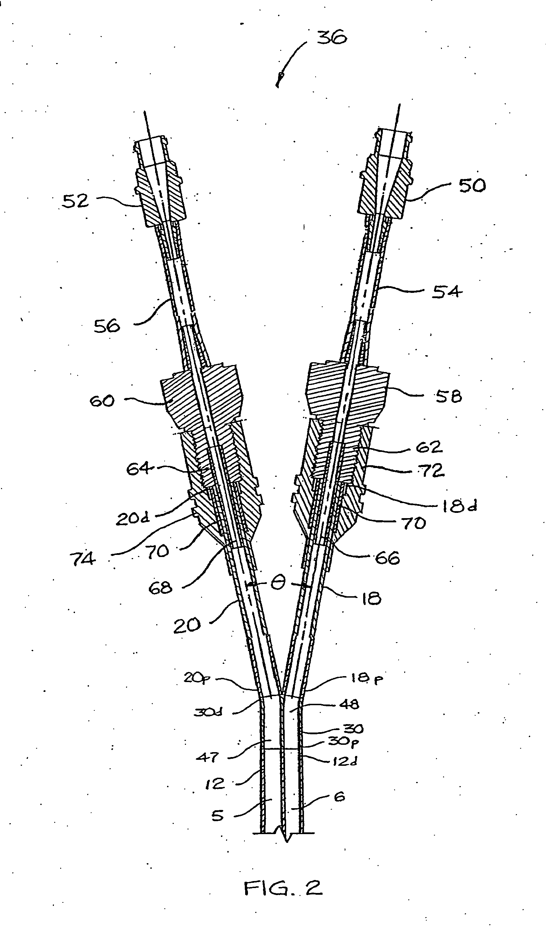

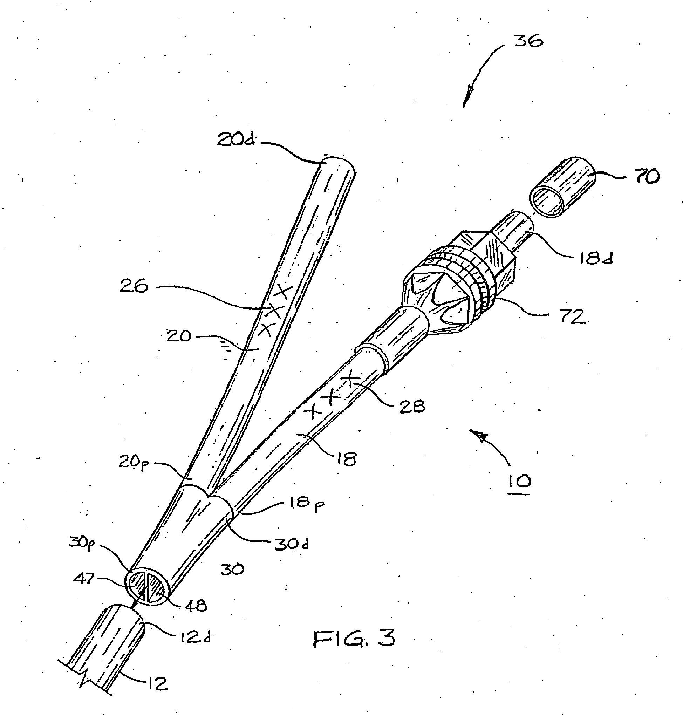

A construction for the distal end 36 of the multi-lumen catheter 10 is shown in Figures 1-3. As shown in Figures 2 and 3, the proximal ends 18p, 20p of the distal extension tubes 18, 20 may be connected to a distal end 12d of the central tube portion 12 by a distal multi-lumen trunk 30. The multiple lumens of the distal trunk 30 correspond in number to the multiple lumens of the central tube portion 12 and the number of distal extension tubes 18, 20. In the illustrated embodiment, the distal trunk 30 includes a venal distal trunk lumen 48, and an arterial distal trunk lumen 47 as shown in Figure 2. As shown in Figure 3, the proximal ends 18p, 20p of the distal extension tubes 18, 20 are connected to the distal end 30d of the distal trunk, thereby forming a substantially Y-shaped junction. The proximal end 30p of the distal trunk 30 is connected to the distal end 12d of the central tube portion 12 as shown in Figure 2, thereby forming a Y-shaped distal end 36. The proximal end 30p of the distal trunk 30 is substantially cylindrical in shape, and is substantially equal in outer diameter to the outer diameter of the central tube portion 12, thereby providing a smooth transition at the juncture between the distal trunk 30 and the central tube portion 12.

As shown in Figure 2, the distal extension tubes are arranged such that an included angle "θ" exists between the longitudinal axes of the tubes 18, 20 in a free state. In a preferred arrangement, the angle "θ" ranges from about 10 degrees to about 30 degrees. The distal extension tubes 18,20 can be arranged, however, so that the angle "θ" is any desired angle. The venal distal trunk lumen 48 is in fluid communication with the venal lumen 6 of the central tube portion 12 and the single lumen of the distal venal extension tube 18. Similarly, the arterial distal trunk lumen 47 is in fluid communication with the arterial lumen 5 of the and the single lumen of the distal arterial extension tube 20.

In an alternative arrangement, the distal extension tubes 18, 20 may be connected directly to the distal end 12d of the central tube portion 12 rather than to an interconnected distal trunk 30 (not shown). In either arrangement, the mating end portions of the distal extension tubes 18,20, the distal end of the central tube portion 12d, and/or the distal trunk 30 are sealably fused together by heat welding or the like such that the fluid communication between the interconnecting lumens of the components is established and maintained and no leakage occurs at the connections.

As shown in Figures 1-3, the catheter 10 also includes selectively attachable connector hubs 72, 74 on the distal ends 18d, 20d of the distal extension tubes 18, 20. As will be described in detail below, the connector hubs 72, 74 are selectively attachable so that the connector hubs 72, 74 can be attached to and removed from the distal end 36 of the catheter 10 after insertion of the proximal end 34 of the catheter 10 into a patient, and after reverse, subcutaneous tunneling of the distal end 36. As shown in Figures 1 and 2, the connector hubs 72, 74 are configured for selective sealable attachment between the distal ends 18d, 20d of the distal extension tubes 18, 20 and legs of a fluid exchange device. The connector hubs. The venal connector hub 74 is selectively attachable to the distal portion 18d of the distal venal extension tube 18, and the arterial connector hub 72 is selectively attachable to the distal portion 20d of the distal arterial extension tube 20.

In one embodient as shown in Figures 1-3, the selectively attachable hubs 72, 74 are connectable with mating compression fittings 58, 60. The compression fittings may include cannulae 66, 68 and threaded male portions 62, 64 that matingly engage the distal extension tubes 18,20 and the connector hubs 72, 74 as shown in Figure 2. When fully engaged, the hubs 72,74 and compression fittings 58, 60 compress compression sleeves 70 about the distal portions 18d, 20d of the distal extension tubes 18, 20, thereby forming sealed connections. The compression fittings 58, 60 may be further connected to luer-type fittings 50, 52 or the like by connector tubes 54, 56. The luer-type fittings 50, 52 may then be connected to corresponding luer-type connection mechanisms on a fluid exchange device 200. For example, the distal ends of the luer-type fittings 50, 52 may include quarter-turn type threads for leak-tight engagement with matching quarter-turn fittings on the venal and arterial legs of a fluid exchange device 200. Other types of known leak-tight selectively attachable connection configurations may also be used.

As shown in Figures 1 and 3, the connector hubs 72, 74 can be selectively attached to the distal ends 18d, 20d of the distal extension tubes 18, 20. This arrangement permits the distal end 36 of the catheter 10 to be subcutaneously reverse tunneled in a patient as described more fully below without interference from the hubs 72, 74. After the distal end of the catheter 10 is subcutaneously reverse tunneled in a patient such that the distal end 36 protrudes outwardly from the patient, the hubs 72, 74 can be backfit over the distal ends of distal extension tubes 18, 20 as shown for hub 72 in Figure 3. The compression sleeves 70 can then be placed over the distal ends 18d, 20d of the extension tubes 18, 20, and the sealed connections can be completed as shown in Figure 2. The catheter 10 can then be connected to a fluid exchange device 200.

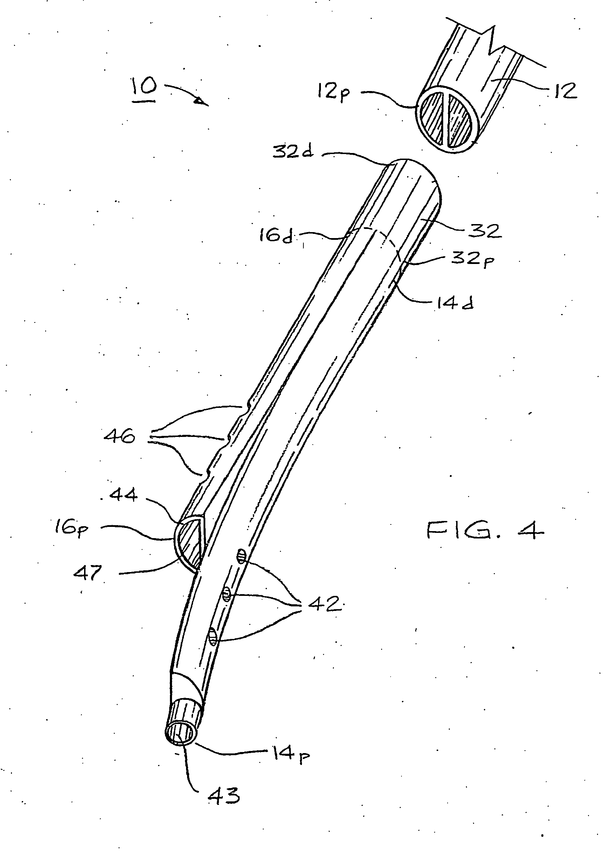

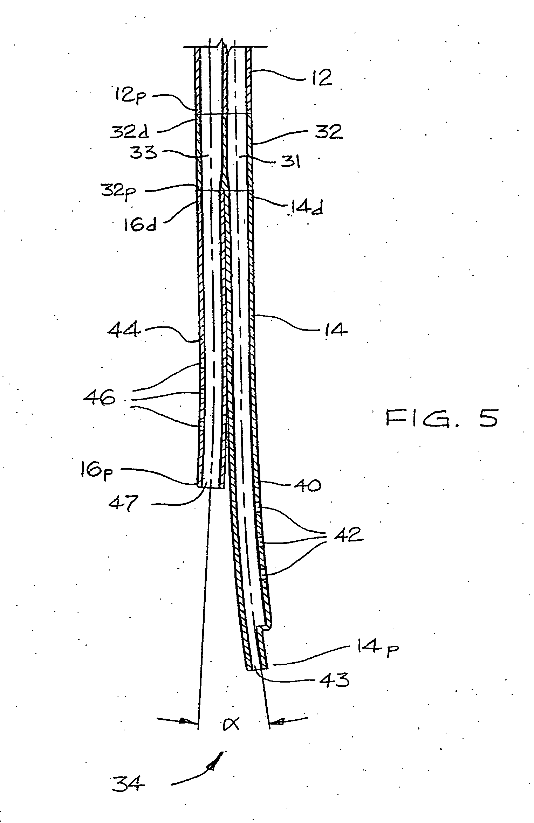

A construction for the proximal end 34 of the catheter 10 is shown in Figures 1,4, and 5. The distal ends 14d, 16d of the proximal extension tubes 14,16 may be connected to a proximal end 12p of the central tube portion 12 by a proximal multi-lumen trunk 32. The lumens of the proximal trunk 32 correspond in number to the multiple lumens of the central tube portion 12 and to the number of proximal extension tubes 14, 16. In the illustrated embodiment, the proximal trunk 32 includes a venal proximal trunk lumen 31, and an arterial proximal trunk lumen 33 as shown in Figure 5. As shown in Figure 4, the distal ends 14d, 16d of the proximal extension tubes 14, 16 are connected to the proximal end 32p of the proximal trunk 32, thereby forming a substantially Y-shaped junction. The distal end 32d of the proximal trunk 32 is connected to the proximal end 12p of the central tube portion 12 as shown in Figure 5, thereby forming a substantially Y-shaped proximal end 34 on the catheter 10. Preferably, the distal end 32d of the proximal trunk 32 is substantially cylindrical in shape, and is substantially equal in outer diameter to the outer diameter of the central tube portion 12, thereby providing a smooth transition at the juncture between the proximal trunk 32 and the central tube portion 12.

As shown in Figure 4, the proximal extension tubes are arranged such that an included angle "α" exists between the longitudinal axes of the tubes 14, 16 in a free state. In a preferred arrangement, the angle "α" is about 5 degrees in a rest position or free state. The distal extension tubes 18, 20 can be arranged, however, so that the angle "α" is any desired angle. The venal proximal trunk lumen 31 is in fluid communication with the venal lumen 6 of the central tube portion 12 and the single lumen of the proximal venal extension tube 14. Similarly, the arterial proximal trunk lumen 33 is in fluid communication with the arterial lumen 5 of the central tube portion 12 and the single lumen of the proximal arterial extension tube 16.

In an alternative arrangement, the proximal extension tubes 14, 16 may be connected directly to the proximal end 12p of the central tube portion 12 rather than to an interconnecting proximal trunk 32 (not shown). In either arrangement, the mating end portions of the proximal extension tubes 14, 16, the proximal end of the central tube portion 12p, and/or the proximal trunk 32 are sealably fused together, such as by heat welding or the like, such that the fluid communication between the interconnected lumens of the components is established and maintained and no leakage occurs at the connections.

As shown in Figures 1, 4, and 5, the proximal arterial extension tube 16 is preferably shorter in length than the proximal venal extension tube 14. For example, the proximal arterial extension tube 16 may be about 4 cm shorter in length than the proximal venal extension tube 14. The resulting longitudinal spacing between the proximal tips 14p and 16p facilitates optimal proximal tip placement in a patient. As shown in Figure 4, the proximal venal extension tube 14 may include an end opening 43 in or near its proximal tip 14p. The proximal venal extension tube 14 may also include one or more transverse openings 42 in its tube wall 40. Similarly, as also shown in Figure 4, the proximal arterial extension tube 16 may include an end opening 47 in or near its proximal tip 16p. The proximal arterial extension tube 16 may also include one or more transverse openings 46 in its tube wall 44. The openings 42, 43, 46, and 47 facilitate fluid flow into or out from the proximal extension tubes 14, 16.

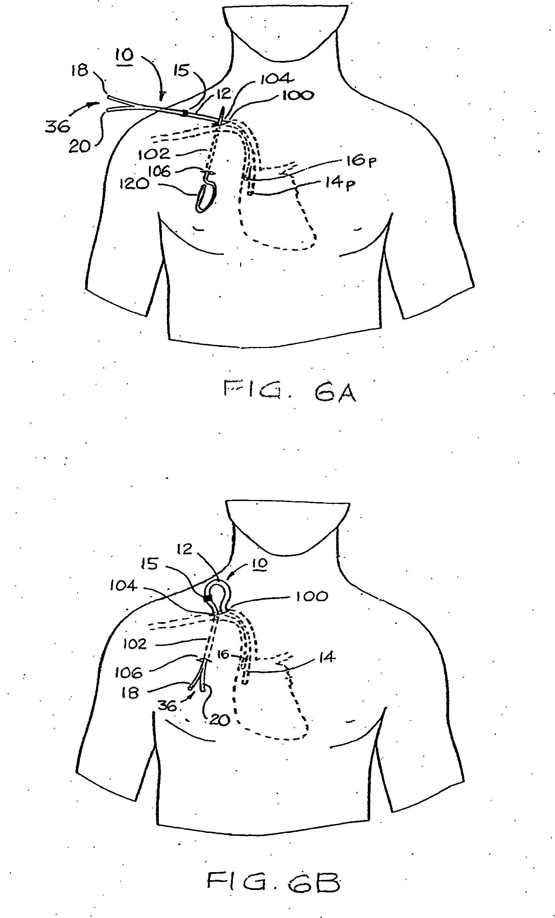

The hubs 72 and 74 are selectively attachable and detachable from the distal end 36 of the catheter 10 to facilitate tunneling the catheter 10 in a patient. A method of installing a catheter 10 in a patient is illustrated in Figures 6A-6D. As shown in Figure 6A, an incision 100 is made in the skin of a patient. The proximal tips 14p, 16p of catheter 10 are inserted through the incision 100 and are placed at desired locations within the patient using conventional techniques, such as the Seldinger technique.

At this stage, the distal end 36 and distal portions of the catheter 10 extend outwardly from the incision 100. A trocar 120 or other suitable instrument is used to form a subcutaneous tunnel 102 having a first end 104, which is preferably coincident with the incision 100, and an opposed second end 106, which is remote from the first end 104, as shown in Figure 6A.

As shown in Figure 6B, the distal end 36 of the catheter 10 is inserted through the first end 104 of the tunnel 102, and the distal end 36 is guided through the tunnel 102 such that the distal end 36 extends out from the tunnel 102 at its second end 106. The distal extension tubes 18, 20 are sufficiently flexible that they may be bundled or clamped together by any suitable means to facilitate passing the Y-shaped distal end 36 of the catheter 10 through the tunnel 102.

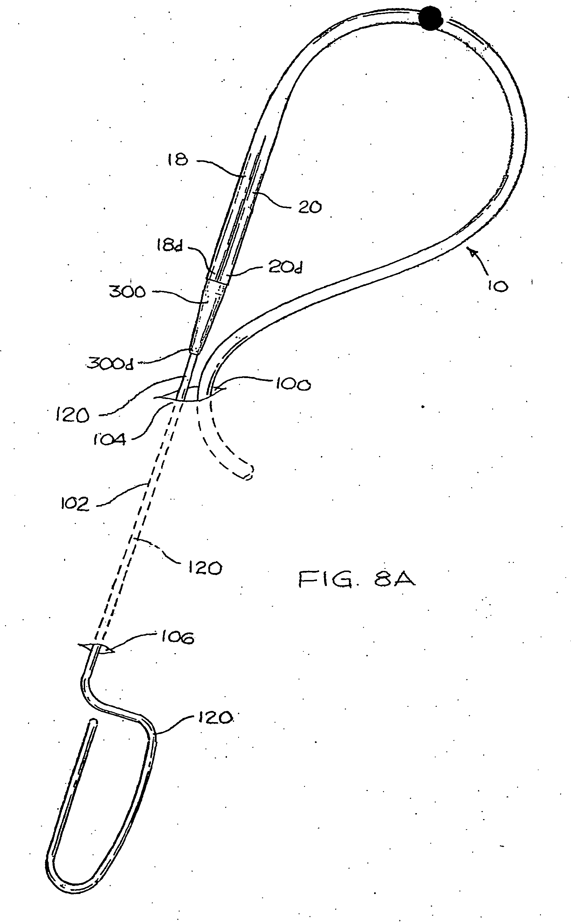

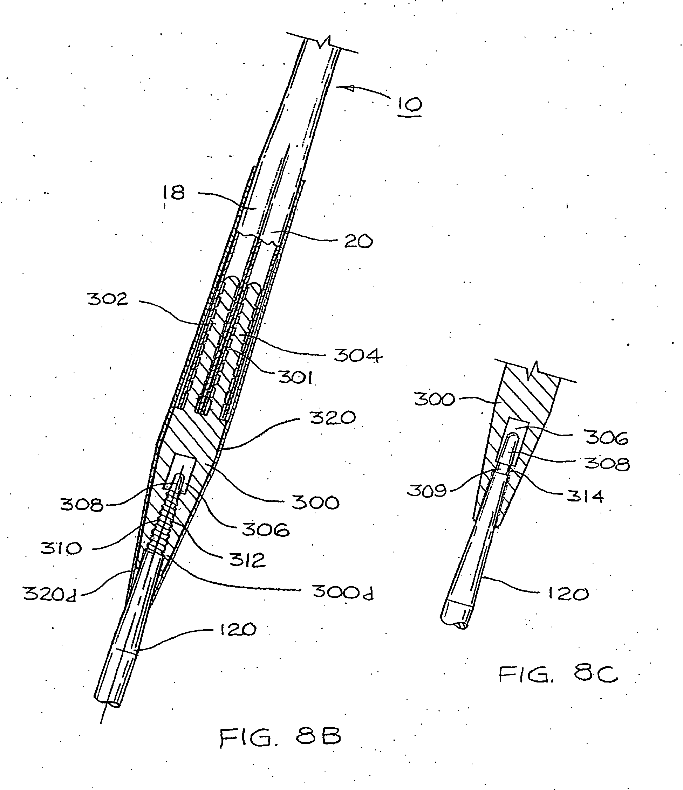

In a preferred arrangement as shown in Figures 8A and 8B, the distal ends 18d, 20d of the distal extension tubes 18, 20 are attached to a connector 300. The proximal end of the connector 300 may include a first tip 302 and a second tip 304 as shown. The tips 302, 304 are insertable into the lumens at the distal ends 18d, 20d of the distal extension tubes 18, 20. The tips 302, 304 preferably include ribs 301 or the like to tightly engage within the distal ends 18d, 20d of the distal extension tubes 18,20 such that the connector 300 is securely but removably attached to the extension tubes 18, 20. When the tips 302, 304 are respectively engaged in the distal extension tubes 18, 20, the connector 300 holds the distal extension tubes 18, 20 in close arrangement as shown so that the distal extension tubes 18, 20 can be simultaneously subcutaneously tunneled in a patient as described below. The distal end of the connector 300d preferably includes a bore 306 which is configured to attachably receive an insertion tip 308 of the trocar 120. The bore 306 may include threads 310 which can be engaged with mating threads 312 on the insertion tip 308 of the trocar 120. Alternatively, the bore 306 may include a collar portion 314 which snaps into a groove 309 on the insertion tip 308 of the trocar 120 as shown in Figure 8C. In this way, the distal end 300d of the connector 300 can be engaged on the insertion tip 308 of the trocar 120 to route the attached distal extension tubes 18, 20 through the subcutaneous tunnel 102 with the trocar 120. Once the distal end 36 of the catheter 10 has been drawn through the tunnel 102, the connector 300 can be disengaged from the distal extension tubes 18, 20.

In order to provide the distal portions of the catheter 10 with a smooth and compact outer profile to facilitate passage of the distal end 36 of the catheter 10 through the tunnel 102, a sheath 320 may be used as shown in Figure 8B. The sheath 320 is placed over at least a portion of the connector 300 and the distal extension tubes 18,20. Preferably, the distal end 320d of the sheath 320 is tapered as shown. The sheath 320 and the distal portions of the catheter 10 can be drawn together through the tunnel 102 with the trocar 120. The sheath 320 is removed from the catheter 10 once the distal portions of the catheter 10 have been drawn through the tunnel 102.

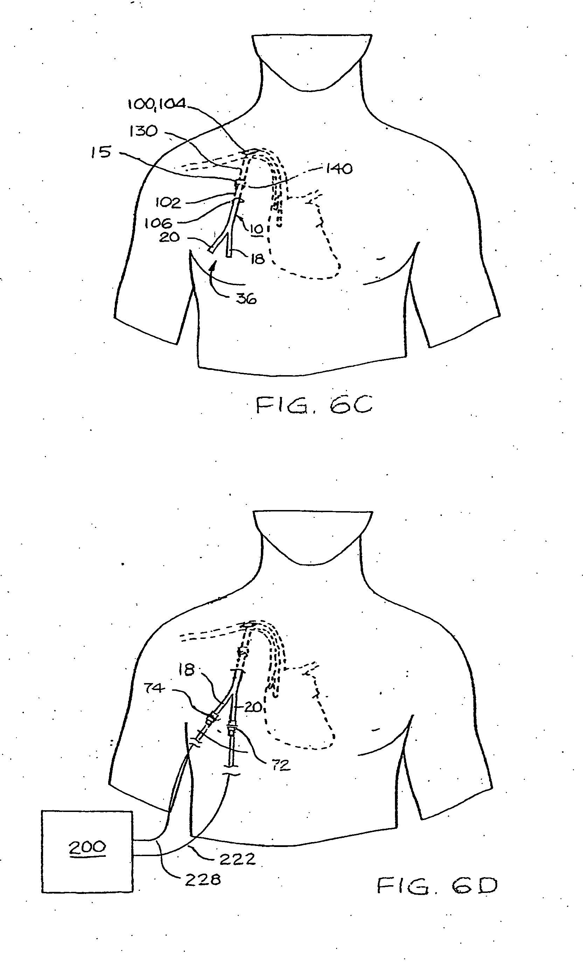

As shown in Figure 6C, the distal end 36 of the catheter 10 is drawn from the second end 104 of the tunnel 102 such that the distal extension tubes 18,20 and at least a portion of the central tube portion 12 extends from the second end 104 and the catheter 10 is fully tunnelled in the patient. The incision 100 and the second end 104 of the tunnel are suitably treated and dressed.

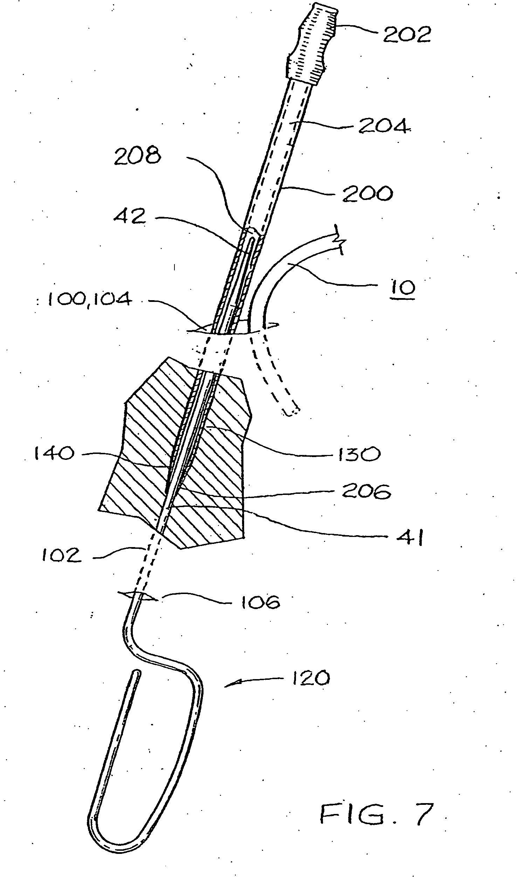

An outer portion of the central tube portion 12 may include a tissue in-growth stabilizing cuff 15, as shown in Figure 1, for stabilizing the inserted catheter 10 in the patient. Referring to Figures 6C and 7, when the catheter 10 includes a stabilizing cuff 15, a portion 130 of tunnel 102 may be dilated to enlarge the width of the tunnel 102 to receive the cuff 15 as the catheter 10 is drawn through the tunnel 102. As shown in Figure 7, the dilated portion 130 of the tunnel is preferably dilated by sliding a sheath dilator 200 over an end 42 and shaft 41 of the trocar 120 when the trocar 120 is positioned in the subcutaneous tunnel 102 as shown in Figure 6A. The sheath dilator 200 preferably includes a hollow bore 208, a tapered leading end 206, a substantially cylindrical portion 204, and a handle 202. The sheath dilator 200 is inserted through the first end 104 of the tunnel 102 and into the tunnel 102 until the tip 206 has been inserted proximate to a cuff seating point 140 in the tunnel 102 to form a dilated portion 130 of the tunnel 102. Once the dilated portion 130 is sufficiently dilated, the sheath dilator 200 is removed from the tunnel 102 and the trocar 120. The catheter 10 is finally positioned in the tunnel 102 when the cuff 15 is seated near an end 140 of the dilated portion 130 of the tunnel 102 as shown in Figure 6C.

As shown in Figure 6D, the catheter 10 is connected to a fluid exchange device 200. The distal end 18d of the distal venal extension tube 18 is selectively attached to a venal leg 224 of the fluid exchange device 200 by connector hub 74. Similarly, the distal end 20d of the distal arterial extension tube 20 is selectively attached to an arterial leg 222 of the fluid exchange device 200 by connector hub 72. As shown in Figure 3, indicia 26 and 28 may be included on the distal extension tubes 18,20 and/or the connector hubs 72, 74 to assist medical personnel in identifying the proper distal extension tube 18 or 20 for connection to a corresponding leg of the fluid exchange device 300. The indicia 26, 28 may be markings, colors, or any other distinctive indicator.

While this invention has been illustrated and described in accordance with a preferred embodiment, it is recognized that variations and changes may be made therein without departing from the invention as set forth in the claims. Certain modifications and improvements will occur to those skilled in the art upon a reading of the forgoing description. For example, while the multi-lumen catheter has been described with reference to a catheter with two lumens, the invention also includes multi-lumen catheters including three or more lumens as required. It should be understood that all such modifications are not contained herein for the sake of conciseness and readability, but are properly within the scope of the following claims.

## Claims

1. A multi-lumen catheter (10) comprising:
(a) a central, elongated, multi-lumen tube portion (12) having a proximal end (12p) and a distal end (12d), the central tube portion (12) having a substantially cylindrical outer shape and being internally segmented into a plurality of lumens (5, 6);
(b) a distal branch portion (36) comprising a plurality of single-lumen distal extension tubes (18, 20), each distal extension tube having a proximal first end (18p, 20p) and a distal second end (18d, 20d), the proximal first end (18p, 20p) of each distal extension tube being permanently connected to the distal end (30d) of a Y-shaped distal trunk (30), the single lumen of each distal extension tube being in fluid communication with one of the plurality of lumens of the central tube portion (12);
(c) a proximal branch portion (34) comprising a plurality of single-lumen proximal extension tubes (14, 16), each proximal extension tube having a distal first end (14d, 16d) and a proximal second end (14p, 16p), the distal first end of each proximal extension tube being connected to the proximal end (12p) of the central tube portion (12) such that the single lumen of each distal extension tube is in fluid communication with one of the plurality of lumens of the central tube portion (12); and
(d) a plurality of selectively attachable connector hubs (72, 74) including venal and arterial connector hubs, each connector hub being configured to be selectively attachable to the distal second end of one of the distal extension tubes and being configured for selective connection to a fluid exchange device,
wherein each lumen of the central tube portion (12) and the lumens of the distal and proximal extension tubes in fluid communication therewith define a flow path through the multi-lumen catheter (10) **characterized in that**
the Y-shaped distal trunk (30) having a proximal end (30p) and a distal end (30d), the proximal end (30p) of the Y-shaped distal trunk (30) being connected to the distal end (12d) of the central tube portion (12), the proximal end (30p) of the Y-shaped distal trunk (30) having a substantially cylindrical outer shape, and the outer diameter of the proximal end (30p) of the Y-shaped distal trunk (30) being substantially equal to the outer diameter of the central tube portion (12).

2. A multi-lumen catheter according to Claim 1, wherein the central tube portion (12) includes two lumens, the distal branch portion (36) includes two single-lumen distal extension tubes (18, 20), and the proximal branch portion (34) includes two proximal extension tubes (14, 16).

3. A multi-lumen catheter according to any of Claims 1 or 2, wherein the central tube portion (12), the distal extension tubes (18, 20), and the proximal extension tubes (14, 16) are comprised of a fusible material, and the distal extension tubes (18, 20) and proximal extension tubes (14, 16) are respectively fused to the distal and proximal ends of the Y-shaped distal trunk (30).

4. A multi-lumen catheter according to any of Claims 1 to 3, wherein the distal extension tubes (18, 20) have a substantially cylindrical outer shape near their distal second ends (18d, 20d).

5. A multi-lumen catheter according to any of Claims 1 to 4, wherein the proximal extension tubes (14, 16) have a substantially D-shaped cross-section over at least a portion of their length.

6. A multi-lumen catheter according to any of Claims 1 to 5, wherein the proximal end (30p) of the Y-shaped distal trunk (30) has a substantially cylindrical outer shape.

7. A multi-lumen catheter according to any of Claims 1 to 6, wherein the proximal extension tubes (14, 16) are substantially parallel to each other in a free state.

8. A multi-lumen catheter according to Claim 1, wherein at least one of the proximal extension tubes (14, 16) is shorter in length than at least one other of the proximal extension tubes (14, 16).

9. A multi-lumen catheter according to any of Claims 1 to 8, further including a stabilizing cuff (15) affixed to an outer portion of the central tube portion (12).

10. A multi-lumen catheter according to any of Claims 1 to 9, wherein the proximal end of each selectively attachable connector hub includes a tube portion (66, 68) configured to be sealably inserted into a portion of a lumen at the distal second end of one of the distal extension tubes (18, 20).

11. A multi-lumen catheter according to Claim 10, wherein the tube portion (66, 68) of each connector hub includes at least one outer circumferential step, barb, or ridge (62, 64) for releasably gripping a portion of a distal extension tube into which the tube portion is inserted.

12. A multi-lumen catheter according to any of Claims 1 to 11, wherein each of the proximal extension tubes (14, 16) includes a tube wall (40, 44), and each of the proximal extension tubes (14, 16) includes at least one opening (42, 46) extending through its tube wall.

13. A multi-lumen catheter according to any of Claims 1 to 12, wherein an external portion of at least one of the distal extension tubes (14, 16) includes indicia (26, 28), the indicia indicating a discrete flow path through the catheter.

14. A multi-lumen catheter according to any of Claims 2 to 13, wherein the longitudinal axes of the two distal extension tubes (18, 20) intersect at an included angle (θ) in a free state, the included angle being in a range from about 10 degrees to about 30 degrees.

15. A multi-lumen catheter according to any of Claims 2 to 14, wherein the two proximal extension tubes (14, 16) have longitudinal axes which intersect at an included angle (α) in a free state, the included angle being at least about 5 degrees.

16. A method of forming a multi-lumen catheter, the method comprising:
permanently attaching a first plurality of single-lumen extension tubes (18, 20) to a distal end (30d) of a Y-shaped distal trunk (30);
permanently attaching a central, elongated, multi-lumen tube portion (12) comprising a plurality of internally segmented lumens (5, 6) to a proximal end (30p) of the Y-shaped distal trunk (30); and
forming at least one opening (42) in a wall (40) of a proximal venal extension tube (14) in fluid communication with one of the internally segmented lumens of the multi-lumen tube portion (12), and forming at least one opening (46) in a wall (44) of an arterial proximal extension tube (16) in fluid communication with the other of the internally segmented lumens of the multi-lumen tube portion (12),
wherein the proximal end (30p) of the Y-shaped distal trunk (30) has a substantially cylindrical outer shape, and the outer diameter of the proximal end (30p) of the Y-shaped distal trunk (30) is substantially equal to the outer diameter of the central, elongated, multi-lumen tube portion (12), and
wherein each single lumen extension tube (18, 20) is in fluid communication with one of the internally segmented lumens of the multi-lumen tube portion (12).

17. A method according to Claim 16, wherein each of the first plurality of single-lumen extension tubes (18, 20) has a longitudinal axes which intersects at an included angle in a free state from the longitudinal axis of the central, elongated, multi-lumen tube portion (12).

18. The method according to any of Claims 16 and 17, wherein the first plurality of single-lumen extension tubes (18, 20) have longitudinal axes which intersect at an included angle (θ) in a free state, the included angle being in a range from about 10 degrees to about 30 degrees.

19. Use of a multi-lumen catheter according to any of Claims 1 to 15 for the manufacture of an apparatus for dialysis treatment.

20. Use of a multi-lumen catheter according to Claim 19, wherein the plurality of single-lumen extension tubes (18, 20) are connected to arterial and venal legs of a fluid exchange device.

21. Use of a multi-lumen catheter according to Claim 19 or 20, wherein the plurality of single-lumen distal extension tubes (18, 20) are connected to the arterial and venal legs of a fluid exchange device with a first connector hub and a second connector hub.

## Patentansprüche

1. Multilumen-Katheter (10), aufweisend:
(a) einen zentralen länglichen Multilumen-Rohrabschnitt (12), der ein proximales Ende (12p) und ein distales Ende (12d) aufweist, wobei der zentrale Rohrabschnitt (12) eine im Wesentlichen zylindrische äußere Gestalt hat und intern in eine Mehrzahl von Lumen (5, 6) segmentiert ist;
(b) einen distalen Verzweigungsabschnitt (36), der eine Mehrzahl von distalen Einzellumen-Verlängerungsrohren (18, 20) aufweist, wobei jedes distale Verlängerungsrohr ein proximales erstes Ende (18p, 20p) und ein distales zweites Ende (18d, 20d) aufweist, wobei das proximale erste Ende (18p, 20p) eines jeden distalen Verlängerungsrohrs in permanenter Weise mit dem distalen Ende (30d) eines Y-förmigen distalen Stammstücks (30) verbunden ist, wobei das Einzellumen eines jeden distalen Verlängerungsrohrs in Fluidkommunikation mit einem von der Mehrzahl von Lumen des zentralen Rohrabschnitts (12) ist;
(c) einen proximalen Verzweigungsabschnitt (34), der eine Mehrzahl von proximalen Einzellumen-Verlängerungsrohren (14, 16) aufweist, wobei jedes proximale Verlängerungsrohr ein distales erstes Ende (14d, 16d) und ein proximales zweites Ende (14p, 16p) aufweist, wobei das distale erste Ende eines jeden proximalen Verlängerungsrohrs mit dem proximalen Ende (12p) des zentralen Rohrabschnitts (12) verbunden ist, derart, dass das Einzellumen eines jeden distalen Verlängerungsrohrs in Fluidkommunikation mit einem von der Mehrzahl von Lumen des zentralen Rohrabschnitts (12) ist; und
(d) eine Mehrzahl von wahlweise anbringbaren Anschlussstücken (72, 74), die venöse und arterielle Anschlussstücke beinhalten, wobei jedes Anschlussstück konfiguriert ist, um wahlweise an dem distalen zweiten Ende eines der distalen Verlängerungsrohre befestigt zu werden, und konfiguriert ist, um wahlweise eine Verbindung zu einer Fluidaustauschvorrichtung herzustellen,
wobei jedes Lumen des zentralen Rohrabschnitts (12) und die Lumen der distalen und proximalen Verlängerungsrohre, die im Fluidkommunikation mit diesen sind, einen Strömungsweg durch den Multilumen-Katheter (10) begrenzen, **dadurch gekennzeichnet, dass**
das Y-förmige distale Stammstück (30) ein proximales Ende (30p) und ein distales Ende (30d) aufweist, wobei das proximale Ende (30p) des Y-förmigen distalen Stammstücks (30) mit dem distalen Ende (12d) des zentralen Rohrabschnitts (12) verbunden ist, wobei das proximale Ende (30p) des Y-förmigen distalen Stammstücks (30) eine im Wesentlichen zylindrische äußere Gestalt hat, und der Außendurchmesser des proximalen Endes (30p) des Y-förmigen distalen Stammstücks (30) im Wesentlichen gleich groß wie der Außendurchmesser des zentralen Rohrabschnitts (12) ist.

2. Multilumen-Katheter nach Anspruch 1, wobei der zentrale Rohrabschnitt (12) zwei Lumen beinhaltet, der distale Verzweigungsabschnitt (36) zwei distale Einzellumen-Verlängerungsrohre (18, 20) beinhaltet, und der proximale Verzweigungsabschnitt (34) zwei proximale Verlängerungsrohre (14, 16) beinhaltet.

3. Multilumen-Katheter nach einem der Ansprüche 1 oder 2, wobei der zentrale Rohrabschnitt (12), die distalen Verlängerungsrohre (18, 20) und die proximalen Verlängerungsrohre (14, 16) aus einem schmelzbaren Material bestehen, und die distalen Verlängerungsrohre (18, 20) und die proximalen Verlängerungsrohre (14, 16) jeweils durch Verschmelzen mit den distalen und proximalen Enden des Y-förmigen distalen Stammstücks (30) verbunden sind.

4. Multilumen-Katheter nach einem der Ansprüche 1 bis 3, wobei die distalen Verlängerungsrohre (18, 20) eine im Wesentlichen zylindrische äußere Gestalt nahe ihrer distalen zweiten Enden (18d, 20d) aufweisen.

5. Multilumen-Katheter nach einem der Ansprüche 1 bis 4, wobei die proximalen Verlängerungsrohre (14, 16) einen im Wesentlichen D-förmigen Querschnitt über zumindest einen Teil ihrer Länge aufweisen.

6. Multilumen-Katheter nach einem der Ansprüche 1 bis 5, wobei das proximale Ende (30p) des Y-förmigen distalen Stammstücks (30) eine im Wesentlichen zylindrische äußere Gestalt hat.

7. Multilumen-Katheter nach einem der Ansprüche 1 bis 6, wobei die proximalen Verlängerungsrohre (14, 16) in freiem Zustand im Wesentlichen parallel zueinander sind.

8. Multilumen-Katheter nach Anspruch 1, wobei zumindest eines der proximalen Verlängerungsrohre (14, 16) von geringerer Länge als mindestens ein anderes der proximalen Verlängerungsrohre (14, 16) ist.

9. Multilumen-Katheter nach einem der Ansprüche 1 bis 8, der weiter eine stabilisierende Manschette (15) aufweist, die an einem äußeren Abschnitt des zentralen Rohrabschnitts (12) befestigt ist.

10. Multilumen-Katheter nach einem der Ansprüche 1 bis 9, wobei das proximale Ende eines jeden wahlweise anbringbaren Anschlussstücks einen Rohrabschnitt (66, 68) beinhaltet, der konfiguriert ist, um dicht abschließend in einen Abschnitt eines Lumens eingesetzt zu werden, und zwar am distalen zweiten Ende von einem der distalen Verlängerungsrohre (18,20).

11. Multilumen-Katheter nach Anspruch 10, wobei der Rohrabschnitt (66, 68) eines jeden Anschlussstücks mindestens eine Stufe, einen Widerhaken oder eine Rippe (62, 64) beinhaltet, die sich am Außenumfang befinden, um einen Abschnitt eines distalen Verlängerungsrohrs, in welchen der Rohrabschnitt eingesetzt wird, lösbar festzuhalten.

12. Multilumen-Katheter nach einem der Ansprüche 1 bis 11, wobei jedes der proximalen Verlängerungsrohre (14, 16) eine Rohrwand (40, 44) beinhaltet, und jedes der proximalen Verlängerungsrohre (14, 16) mindestens eine Öffnung (42, 46) beinhaltet, die sich durch seine Rohrwand hindurch erstreckt.

13. Multilumen-Katheter nach einem der Ansprüche 1 bis 12, wobei ein externer Abschnitt von mindestens einem der distalen Verlängerungsrohre (14, 16) Markierungen (26, 28) beinhaltet, wobei die Markierungen einen separaten Strömungsweg durch den Katheter bezeichnen.

14. Multilumen-Katheter nach einem der Ansprüche 2 bis 13, wobei die Längsachsen der zwei distalen Verlängerungsrohre (18, 20) sich in freiem Zustand unter einem eingeschlossenen Winkel (θ) kreuzen, wobei der eingeschlossene Winkel in einem Bereich von ca. 10° bis ca. 30° liegt.

15. Multilumen-Katheter nach einem der Ansprüche 2 bis 14, wobei die zwei proximalen Verlängerungsrohre (14, 16) Längsachsen aufweisen, die sich in freiem Zustand unter einem eingeschlossenen Winkel (α) kreuzen, wobei der eingeschlossene Winkel mindestens ca. 5° beträgt.

16. Verfahren zur Ausbildung eines Multilumen-Katheters, wobei das Verfahren umfasst:
permanentes Befestigen einer ersten Mehrzahl von Einzellumen-Verlängerungsrohren (18, 20) an einem distalen Ende (30d) eines Y-förmigen distalen Stammstücks (30);
permanentes Befestigen eines zentralen länglichen Multilumen-Rohrabschnitts (12), der eine Mehrzahl von intern segmentierten Lumen (5, 6) aufweist, an einem proximalen Ende (30p) des Y-förmigen distalen Stammstücks (30); und
Ausbilden mindestens einer Öffnung (42) in einer Wand (40) eines proximalen venösen Verlängerungsrohrs (14), das sich in Fluidkommunikaton mit einem der intern segmentierten Lumen des Multilumen-Rohrabschnitts (12) befindet, und Ausbilden mindestens einer Öffnung (46) in einer Wand (44) eines arteriellen proximalen Verlängerungsrohrs (16), das sich in Fluidkommunikation mit dem anderen der intern segmentierten Lumen des Multilumen-Rohrabschnitts (12) befindet,
wobei das proximale Ende (30p) des Y-förmigen distalen Stammstücks (30) eine im Wesentlichen zylindrische äußere Gestalt hat, und der Außendurchmesser des proximalen Endes (30p) des Y-förmigen distalen Stammstücks (30) im Wesentlichen gleich groß wie der Außendurchmesser des zentralen länglichen Multilumen-Rohrabschnitts (12) ist, und
wobei jedes Einzellumen-Verlängerungsrohr (18, 20) in Fluidkommunikation mit einem der intern segmentierten Lumen des Multilumen-Rohrabschnitts (12) ist.

17. Verfahren nach Anspruch 16, wobei jedes der ersten Mehrzahl von Einzellumen-Verlängerungsrohren (18, 20) eine Längsachse aufweist, wobei sich die Längsachsen in freiem Zustand unter einem eingeschlossenen Winkel kreuzen, und zwar von der Längsachse den zentralen länglichen Multilumen-Rohrabschnitts (12).

18. Verfahren nach einem der Ansprüche 16 und 17, wobei die erste Mehrzahl von Einzellumen-Verlängerungsrohren (18, 20) Längsachsen aufweisen, die sich in freiem Zustand unter einem eingeschlossenen Winkel (θ) kreuzen, wobei der eingeschlossene Winkel in einem Bereich von ca. 10° bis ca. 30° liegt.

19. Verwendung eines Multilumen-Katheters nach einem der Ansprüche 1 bis 15 zur Herstellung einer Vorrichtung zur Dialysebehandlung.

20. Verwendung eines Multilumen-Katheters nach Anspruch 19, wobei die Mehrzahl von Einzellumen-Verlängerungsrohren (18, 20) mit arteriellen und venösen Strängen einer Fluidaustauschvorrichtung verbunden sind.

21. Verwendung eines Multilumen-Katheters nach Anspruch 19 oder 20, wobei die Mehrzahl von Einzellumen-Verlängerungsrohren (18, 20) mit arteriellen und venösen Strängen einer Fluidaustauschvorrichtung mit einem ersten Anschlussstück und einem zweiten Anschlussstück verbunden sind.

## Revendications

1. Cathéter à lumières multiples (10) comprenant :
(a) une partie de tube centrale, allongée, à lumières multiples (12) ayant une extrémité proximale (12p) et une extrémité distale (12d), la partie de tube centrale (12) ayant une forme externe sensiblement cylindrique et étant intérieurement segmentée en une pluralité de lumières (5, 6) ;
(b) une partie de branche distale (36) comprenant une pluralité de tubes de prolongement distaux à lumière unique (18, 20), chaque tube de prolongement distal ayant une première extrémité proximale (18p, 20p) et une seconde extrémité distale (18d, 20d), la première extrémité proximale (18p, 20p) de chaque tube de prolongement distal étant raccordée en permanence à l'extrémité distale (30d) d'un tronc distal en forme de Y (30), la lumière unique de chaque tube de prolongement distal étant en communication fluidique avec l'une de la pluralité de lumières de la partie de tube central (12) ;
(c) une partie de branche proximale (34) comprenant une pluralité de tubes de prolongement proximaux à lumière unique (14, 16), chaque tube de prolongement proximal ayant une première extrémité distale (14d, 16d) et une seconde extrémité proximale (14p, 16p), la première extrémité distale de chaque tube de prolongement proximal étant raccordée à l'extrémité proximale (12p) de la partie de tube centrale (12) de telle sorte que la lumière unique de chaque tube de prolongement distal est en communication fluidique avec l'une de la pluralité de lumières de la partie de tube centrale (12) ; et
(d) une pluralité d'embouts de raccordement pouvant être sélectivement attachés (72, 74) incluant des embouts de raccordement vénal et artériel, chaque embout de raccordement étant configuré pour sélectivement s'attacher à la seconde extrémité distale de l'un des tubes de prolongement distaux et étant configuré pour être sélectivement raccordé à un dispositif d'échange de fluide,
dans lequel chaque lumière de la partie de tube centrale (12) et les lumières des tubes de prolongement distaux et proximaux en communication fluidique avec celle-ci définissent un trajet d'écoulement à travers le cathéter à lumières multiples (10) **caractérisé en ce que** le tronc distal en forme de Y (30) a une extrémité proximale (30p) et une extrémité distale (30d), l'extrémité proximale (30p) du tronc distal en forme de Y (30) étant raccordée à l'extrémité distale (12d) de la partie de tube centrale (12), l'extrémité proximale (30p) du tronc distal en forme de Y (30) ayant une forme externe sensiblement cylindrique, et le diamètre externe de l'extrémité proximale (30p) du tronc distal en forme de Y (30) étant sensiblement égal au diamètre externe de la partie de tube centrale (12).

2. Cathéter à lumières multiples selon la revendication 1, dans lequel la partie de tube centrale (12) inclut deux lumières, la partie formant branche distale (36) inclut deux tubes de prolongement distaux à lumière unique (18, 20), et la partie de branche proximale (34) inclut deux tubes de prolongement proximaux (14, 16).

3. Cathéter à lumières multiples selon l'une quelconque des revendications 1 et 2, dans lequel la partie de tube centrale (12), les tubes de prolongement distaux (18, 20) et les tubes de prolongement proximaux (14, 16) sont constitués d'un matériau fusible, et les tubes de prolongement distaux (18, 20) et les tubes de prolongement proximaux (14, 16) sont respectivement fusionnés aux extrémités distale et proximale du tronc distal en forme de Y (30).

4. Cathéter à lumières multiples selon l'une quelconque des revendications 1 à 3, dans lequel les tubes de prolongement distaux (18, 20) ont une forme externe sensiblement cylindrique à proximité de leurs secondes extrémités distales (18d, 20d).

5. Cathéter à lumières multiples selon l'une quelconque des revendications 1 à 4, dans lequel les tubes de prolongement proximaux (14, 16) ont une section transversale sensiblement en forme de D sur au moins une partie de leur longueur.

6. Cathéter à lumières multiples selon l'une quelconque des revendications 1 à 5, dans lequel l'extrémité proximale (30p) du tronc distal en forme de Y (30) a une forme externe sensiblement cylindrique.

7. Cathéter à lumières multiples selon l'une quelconque des revendications 1 à 6, dans lequel les tubes de prolongement proximaux (14, 16) sont sensiblement parallèles l'un à l'autre dans un état libre.

8. Cathéter à lumières multiples selon la revendication 1, dans lequel au moins l'un des tubes de prolongement proximaux (14, 16) est plus court en longueur que l'au moins un autre des tubes de prolongement proximaux (14, 16).

9. Cathéter à lumières multiples selon l'une quelconque des revendications 1 à 8, comprenant en outre un ballonnet de stabilisation (15) fixé à une partie externe du partie de tube centrale (12).

10. Cathéter à lumières multiples selon l'une quelconque des revendications 1 à 9, dans lequel l'extrémité proximale de chaque embout de raccordement pouvant être sélectivement fixé inclut une partie de tube (66, 68) configurée pour être insérée de manière étanche dans une partie d'une lumière à la seconde extrémité distale de l'un des tubes de prolongement distaux (18, 20).

11. Cathéter à lumières multiples selon la revendication 10, dans lequel la partie de tube (66, 68) de chaque embout de raccordement inclut au moins une marche, un barbillon ou une arête circonférentiel(le) externe (62, 64) pour se mettre en prise de manière détachable avec une partie d'un tube de prolongement distal dans laquelle est insérée la partie de tube.

12. Cathéter à lumières multiples selon l'une quelconque des revendications 1 à 11, dans lequel chacun des tubes de prolongement proximaux (14, 16) inclut une paroi de tube (40, 44) et chacun des tubes de prolongement proximaux (14, 16) inclut au moins une ouverture (42, 46) s'étendant à travers sa paroi de tube.

13. Cathéter à lumières multiples selon l'une quelconque des revendications 1 à 12, dans lequel une partie externe d'au moins l'un des tubes de prolongement distaux (14, 16) inclut des repères (26, 28), les repères indiquant un trajet d'écoulement discret à travers le cathéter.

14. Cathéter à lumières multiples selon l'une quelconque des revendications 2 à 13, dans lequel les axes longitudinaux des deux tubes de prolongement distaux (18, 20) se coupent à un angle inclus (θ) dans un état libre, l'angle inclus étant dans une plage d'environ 10 degrés à environ 30 degrés.

15. Cathéter à lumières multiples selon l'une quelconque des revendications 2 à 14, dans lequel les deux tubes de prolongement proximaux (14, 16) ont des axes longitudinaux qui se coupent à un angle inclus (α) dans un état libre, l'angle inclus étant d'au moins environ 5 degrés.

16. Procédé de formation d'un cathéter à lumières multiples, le procédé comprenant :
la fixation permanente d'une première pluralité de tubes de prolongement à lumière unique (18, 20) à une extrémité distale (30d) d'un tronc distal en forme de Y (30) ;
la fixation permanente d'une partie de tube centrale, allongée, à lumières multiples (12) comprenant une pluralité de lumières segmentées intérieures (5, 6) à une extrémité proximale (30p) du tronc distal en forme de Y (30) ; et
la formation d'au moins une ouverture (42) dans une paroi (40) d'un tube de prolongement vénal proximal (14) en communication fluidique avec l'une des lumières segmentées intérieures de la partie de tube à lumières multiples (12), et la formation d'au moins une ouverture (46) dans une paroi (44) d'un tube de prolongement proximal artériel (16) en communication fluidique avec l'autre des lumières segmentées intérieures de la partie de tube à lumières multiples (12),
dans lequel l'extrémité proximale (30p) du tronc distal en forme de Y (30) a une forme externe sensiblement cylindrique, et le diamètre externe de l'extrémité proximale (30p) du tronc distal en forme de Y (30) est sensiblement égal au diamètre externe de la partie de tube centrale, allongée, à lumière multiples (12), et
dans lequel chaque tube de prolongement à lumière unique (18, 20) est en communication fluidique avec l'une des lumières segmentées intérieures de la partie de tube à lumières multiples (12).

17. Procédé selon la revendication 16, dans lequel la première pluralité de tubes de prolongement à lumière unique (18, 20) a un axe longitudinal qui coupe à un angle inclus dans un état libre l'axe longitudinal de la partie de tube centrale, allongée, à lumières multiples (12).

18. Procédé selon l'une quelconque des revendications 16 et 17, dans lequel la première pluralité de tubes de prolongement à lumière unique (18, 20) ont des axes longitudinaux qui se coupent à un angle inclus (θ) dans un état libre, l'angle inclus étant dans une plage d'environ 10 degrés à environ 30 degrés.

19. Utilisation d'un cathéter à lumières multiples selon l'une quelconque des revendications 1 à 15 pour la fabrication d'un appareil de traitement de dialyse.

20. Utilisation d'un cathéter à lumières multiples selon la revendication 19, dans lequel la pluralité de tubes de prolongement à lumière unique (18, 20) sont raccordés aux branches artérielle et vénale d'un dispositif d'échange de fluide.

21. Utilisation d'un cathéter à lumières multiples selon la revendication 19 ou 20, dans lequel la pluralité de tubes de prolongement distaux à lumière unique (18, 20) sont raccordés aux branches artérielle et vénale d'un dispositif d'échange de fluide avec un premier embout de raccordement et un second embout de raccordement.
